# EUROPÄISCHE PATENTSCHRIFT

(11) **EP 3 629 970 B1**
(45) Veröffentlichungstag und Bekanntmachung des Hinweises auf die Patenterteilung: **10.04.2024**
(21) Anmeldenummer: 18726945.1
(22) Anmeldetag: 14.05.2018
(51) Int. Cl.: A61B 34/00, A61B 90/50, A61B 90/00, A61B 34/20

(54) **PASSIVER CHIRURGISCHER MANIPULATOR MIT HANDGEHALTENER ANTRIEBSEINHEIT**
PASSIVE SURGICAL MANIPULATOR WITH HAND-HELD DRIVE UNIT
MANIPULATEUR CHIRURGICAL PASSIF MUNI D'UNE UNITÉ D'ENTRAÎNEMENT PORTATIVE

(30) Priorität: 23.05.2017 DE 102017111289
(43) Veröffentlichungstag der Anmeldung: 08.04.2020
(73) Patentinhaber: Brainlab Robotics GmbH, 81829 München (DE)
(72) Erfinder: KRINNINGER, Maximilian, 82234 Wessling-Oberpfaffenhofen (DE); NOWATSCHIN, Stephan, 81677 München (DE)
(74) Vertreter: Eisenführ Speiser
(86) Internationale Anmeldenummer: PCT/EP2018/062331
(87) Internationale Veröffentlichungsnummer: WO 2018/215226

(56) Entgegenhaltungen:
- WO-A1-2005/030074
- WO-A1-2014/198784
- WO-A1-2016/209769
- DE-A1-102014 016 823
- US-A1- 2004 092 912

## Beschreibung

Die Erfindung betrifft einen passiven chirurgischen Manipulator zum Halten und Positionieren eines chirurgischen Instruments, mit einem Rahmen einer mindestens einen Lenker umfassenden ersten Lenkeranordnung, welche den Rahmen mit einem dem Instrument zugeordneten ersten Gelenkpunkt gelenkig verbindet, und einer mindestens einen Lenker umfassenden zweiten Lenkeranordnung, welche den Rahmen mit einem dem Instrument zugeordneten zweiten Gelenkpunkt gelenkig verbindet, wobei die beiden Lenkeranordnungen so ausgebildet sind, dass der erste Gelenkpunkt in einer ersten Bewegungsebene und der zweite Gelenkpunkt in einer zweiten Bewegungsebene bewegbar ist, wobei die erste Lenkeranordnung an ersten und zweiten Stelleinrichtungen mit dem Rahmen verbunden ist, und die zweite Lenkeranordnung an dritten und vierten Stelleinrichtungen mit dem Rahmen verbunden ist.

Offenbart ist ferner eine handgehaltene Antriebseinheit zur Verwendung mit einem passiven chirurgischen Manipulator nach der vorstehend genannten Art. Weiterhin betrifft die Erfindung ein chirurgisches System umfassend wenigstens einen Manipulator nach der vorstehenden Art sowie eine Antriebseinheit nach der vorstehenden Art. Die Erfindung betrifft auch ein nicht-chirurgisches Verfahren zum Einstellen einer Sollposition von zwei Gelenkpunkten eines passiven chirurgischen Manipulators nach der vorstehend genannten Art.

Eine solche chirurgische Manipulatorvorrichtung ist beispielsweise aus EP 1 658 016 B1 bekannt. Die dort offenbarte Vorrichtung ist dazu vorgesehen, ein Endoskop zu halten und ist grundsätzlich motorlos ausgebildet. Die Vorrichtung weist einen Rahmen auf, mit einer mindestens zwei Lenker umfassenden Lenkeranordnung, welche den Rahmen mit einem dem Instrument zugeordneten ersten Gelenkpunkt gelenkig verbindet, und mit einer mindestens zwei Lenker umfassenden zweiten Lenkeranordnung, welche den Rahmen mit einem dem ersten Instrument zugeordneten zweiten Gelenkpunkt gelenkig verbindet, wobei die Lenker der ersten Lenkeranordnung relativ zueinander und relativ zum Rahmen um Schwenkachsen schwenkbar gelagert sind und die Schwenkachsen parallel zueinander verlaufen und wobei die beiden Lenkeranordnungen so ausgebildet sind, das der erste Gelenkpunkt in einer ersten Bewegungsebene und der zweite Gelenkpunkt in einer zweiten Bewegungsebene bewegbar ist, wobei die erste Bewegungsebene relativ zur zweiten Bewegungsebene bewegbar ist. Dies wird bei EP 1 658 016 dazu genutzt, eine sich ändernde Distanz der ersten und zweiten Gelenkpunkte bei unterschiedlicher Verschwenkung bzw. Bewegung der ersten und der zweiten Lenkeranordnungen auszugleichen. Dies ist insbesondere deshalb erforderlich, da das Instrument direkt mit den Gelenkpunkten verbunden ist. Nachteilig an einem solchen Gerät ist insbesondere, dass die Anbindung des Rahmens an ein Stativ schwierig ist, da auch der Rahmen die Bewegung der Ebene erlauben muss.

Die chirurgische Manipulatorvorrichtung der vorliegenden Erfindung soll insbesondere an einem Haltearm aufgenommen werden, wie in DE 10 2014 016 823 A1, DE 10 2014 016 824 A1, DE 10 2015 104 810 A1, DE 10 2015 104 819 A1 und EP 3 130 305 A1 beschrieben. Deren Offenbarungsgehalt in Bezug auf den dort beschriebenen Haltearm wird vollumfänglich hierin mit einbezogen.

WO2014/198784 A1 offenbart einen weiteren Manipulator nach dem Stand der Technik.

Aufgabe der vorliegenden Erfindung ist es eine einfache Manipulatorvorrichtung, eine handgehaltene Antriebseinheit, ein System und ein Verfahren anzugeben, mit denen eine einfache Positionierung eines chirurgischen Instruments möglich ist.

Die Erfindung löst die Aufgabe bei einem passiven chirurgischen Manipulator der eingangs genannten Art dadurch, dass die erste Stelleinrichtung eine erste Schnittstelle, die zweite Stelleinrichtung eine zweite Schnittstelle, die dritte Stelleinrichtung eine dritte Schnittstelle und die vierte Stelleinrichtung eine vierte Schnittstelle zur temporären Aufnahme einer korrespondierenden Schnittstelle einer handgehaltenen Antriebseinheit aufweist, sodass die handgehaltene Antriebseinheit temporär und sequenziell mit den ersten, zweiten, dritten und vierten Schnittstellen zum Übertragen einer Stellbewegung kuppelbar ist, um die ersten und zweiten Gelenkpunkte zu bewegen und in einer vorbestimmten Sollposition zu positionieren.

Hiermit ist ein chirurgischer Manipulator geschaffen, der selbst über keine eigene Antriebseinheit verfügt und somit einfach aufgebaut und kostengünstig herzustellen ist. Vielmehr ist es möglich, dass der komplette Antrieb über eine einzige Antriebseinheit erfolgt, nämlich über die handgehaltene Antriebseinheit, die temporär und sequenziell mit den ersten, zweiten, dritten und vierten Schnittstellen koppelbar ist. Es ist also nur ein einziger Antriebsmotor erforderlich, der in der handgehaltenen Antriebseinheit untergebracht wird, um die ersten und zweiten Lenkeranordnungen zu verstellen.

Die ersten und zweiten Lenkeranordnungen sind vorzugsweise selbsthemmend ausgebildet. Hierdurch wird erreicht, dass auch nach Entfernen der handgehaltenen Antriebseinheit die Position der Lenkeranordnung erhalten wird.

In einer weiteren bevorzugten Ausführungsform weist der chirurgische Manipulator einen Befestigungsabschnitt zum Befestigen des Manipulators an einem chirurgischen Haltearm auf. Der chirurgische Haltearm ist insbesondere als robotischer Haltearm, oder Stativ ausgebildet und vorzugsweise wie in DE 10 2014 016 824 A1, DE 10 2014 016 823 A1 und DE 10 2015 104 819 A1 offenbart ausgebildet. Der Offenbarungsgehalt dieser Druckschriften wird betreffend den Haltearm vollumfänglich hierin mit einbezogen.

In einer weiteren bevorzugten Ausgestaltung der Erfindung ist vorgesehen, dass die Schnittstellen, mit denen die handgehaltene Antriebseinheit temporär und sequenziell koppelbar ist, örtlich voneinander getrennt sind. Hierdurch ist eine intuitive Bedienung des chirurgischen Manipulators möglich und der Bediener erkennt aufgrund der Konstruktion der Kinematik ohne Weiteres, an welcher der Schnittstellen er die Antriebseinheit koppeln muss, um die ersten und zweiten Lenkeranordnungen zu verstellen.

In einer weiteren bevorzugten Ausführungsform sind die Schnittstellen an einer gemeinsamen Kupplungsausnehmung ausgebildet. In dieser Kupplungsausnehmung sind dann vorzugsweise vier verschiedene Abschnitte ausgebildet, die die vier Schnittstellen bilden. Diese können beispielsweise axial hintereinander gelagert angeordnet sein. Beispielsweise ist denkbar, dass jede Schnittstelle von einer Abtriebsscheibe gebildet wird, die ein zentrales Durchgangsloch aufweist, sodass beim Einsetzen der handgehaltenen Antriebseinheit diese mit allen vier Abtriebsscheiben in Kontakt kommt und jede separat antreiben kann. Auf diese Weise ist die Bedienung weiter vereinfacht, da ein Bediener die handgehaltene Antriebseinheit nicht an verschiedenen Positionen des Manipulators einsetzen muss, sondern nur an der gemeinsamen Kupplungsausnehmung.

Bevorzugt ist hierbei, dass die Schnittstellen für eine formschlüssige Aufnahme der Antriebseinheit und zur formschlüssigen Übertragung der Stellbewegung ausgebildet sind. Durch eine formschlüssige Aufnahme der Antriebseinheit ist eine definierte Position der Antriebseinheit in Bezug auf die Schnittstellen erreicht. Eine formschlüssige Übertragung der Stellbewegung resultiert in einer definierten Stellbewegungsübertragung, die schlupffrei ist.

Alternativ dazu sind die Schnittstellen für eine formschlüssige Aufnahme der Antriebseinheit und zur kraftschlüssigen Übertragung der Stellbewegung ausgebildet. Insbesondere weist jede Schnittstelle gemäß dieser Ausführungsform ein ferromagnetisches Kupplungselement auf. Das heißt, ein Abtriebsabschnitt der handgehaltenen Antriebseinheit und ein entsprechendes Kupplungselement der Schnittstelle werden durch magnetische Kraft gekoppelt. Ein Vorteil hierbei liegt darin, dass beim Kuppeln selbst keine bestimmte Position eingenommen werden muss, sondern beispielsweise jede Art von Drehstellung ohne eine Teilung gekuppelt werden kann. Weiterhin können so glatte Oberflächen bereitgestellt werden, die aus hygienischen Gründen im chirurgischen Bereich bevorzugt sind.

Vorzugsweise weist der chirurgische Manipulator wenigstens einen Sensor zur Ermittlung einer Kraft auf, die auf den ersten und/oder zweiten Gelenkpunkt wirkt. Hierdurch ist es möglich, eine Kraft, die auf ein aufgenommenes Instrument wirkt, zu bestimmen. Dies kann beispielsweise dazu eingesetzt werden, zu ermitteln, ob ein Patient bei einer Bewegung der ersten und zweiten Lenkeranordnung falsch belastet wird. So ist es beispielsweise denkbar, dass ein Bediener eine Fehlbedienung vornimmt und die Lenkeranordnungen auf unnütze Weise verstellt. In diesem Fall kann durch die Ermittlung der Kraft an den Gelenkpunkten eine Rückkopplung gegeben werden, die den Bediener der Manipulators warnt. Hierdurch ist insbesondere die Sicherheit verbessert.

Gemäß einer weiteren bevorzugten Ausgestaltung weist der Manipulator eine Steuereinheit mit einem Prozessor und Softwaremitteln auf, die dazu ausgebildet ist, basierend auf einer vorbestimmen Sollstellung des ersten und zweiten Gelenkpunkts eine an jeder Schnittstelle erforderliche Stellbewegung zum Erreichen der Sollstellung zu ermitteln. Vorzugsweise wird hierbei eine Pose der ersten und zweiten Lenkeranordnungen ermittelt. Besonders bevorzugt ist die Steuereinheit dazu eingerichtet, Signale, die die ermittelten Stellbewegungen repräsentieren, an der handgehaltenen Antriebseinheit oder einem Peripheriegerät bereitzustellen. Alternativ dazu ist die handgehaltene Antriebseinheit selbst in der Lage, basierend auf einer vorbestimmten Sollstellung des ersten und zweiten Gelenkpunkts, eine an jeder Schnittstelle erforderliche Stellbewegung zum Erreichen der Sollstellung zu ermitteln. Wird dies durch die Steuereinheit des Manipulators erreicht, werden die Signale, die die ermittelten Stellbewegungen repräsentieren, vorzugsweise an die handgehaltene Antriebseinheit oder ein Peripheriegerät gesendet. Dies kann beispielsweise drahtlos erfolgen. Ein Peripheriegerät kann insbesondere ein Haltearm sein, an dem der Manipulator aufgenommen ist, oder ein OP-Navigationssystem. Es ist beispielsweise denkbar, dass ein OP-Navigationssystem eine vorbestimmte Sollstellung an den Manipulator sendet, der Manipulator basierend auf der empfangenen vorbestimmten Sollstellung die entsprechenden Stellbewegungen ermittelt, diese dann an die handgehaltene Antriebseinheit übermittelt und die handgehaltene Antriebseinheit die Stellbewegungen dann ausführt, wenn diese mit der entsprechenden Schnittstelle gekoppelt ist.

Bevorzugt weist jede Schnittstelle eine Anzeigeeinheit auf, die dazu eingerichtet ist, ein Erreichen der Sollstellung anzuzeigen. Vorzugsweise zeigt die Anzeigeeinheit auch eine Sequenz der Kupplung mit der handgehaltenen Antriebseinheit an. Vorzugsweise zeigt die Anzeigeeinheit an, dass die handgehaltene Antriebseinheit mit der entsprechenden Schnittstelle zu koppeln ist. Beispielsweise ist denkbar, dass die Anzeigeeinheit eine LED-Beleuchtung aufweist, und die LED-Beleuchtung der ersten Schnittstelle in einer ersten Farbe aufleuchtet, wenn die erste Schnittstelle mit der handgehaltenen Antriebseinheit zu koppeln ist. Nach Erreichen der Sollstellung leuchtet dann die LED-Leuchte der ersten Schnittstelle in einer zweiten Farbe auf, um das Erreichen der Sollstellung anzuzeigen. Anschließend leuchtet beispielsweise die LED-Leuchte der zweiten Schnittstelle in der ersten Farbe auf, um das Anfordern der Kopplung der handgehaltenen Antriebseinheit mit der zweiten Schnittstelle anzuzeigen. Entsprechendes gilt auch für die weiteren Schnittstellen.

Vorzugsweise weist der Manipulator eine oder mehrere Referenzen für ein chirurgisches Navigationssystem auf. Auf diese Weise kann der Manipulator in ein chirurgisches Navigationssystem eingebunden werden und von diesem erkannt werden.

Gemäß einer weiteren bevorzugten Ausführungsform sind an den Schnittstellen Markierungen angeordnet, die durch eine entsprechende Leseeinheit abtastbar sind zur Ermittlung einer Stellung der ersten und zweiten Gelenkpunkte. Die Markierungen sind vorzugsweise so angeordnet, dass diese beim Übertragen einer Stellbewegung von der handgehaltenen Antriebseinheit auf den Manipulator mitbewegt werden. Anhand dieser Markierungen ist dann die Stellung der ersten und zweiten Lenkeranordnungen ablesbar und damit die Stellung der ersten und zweiten Gelenkpunkte ermittelbar.

Gemäß einer weiteren bevorzugten Ausführungsform ist vorgesehen, dass der Manipulator mittels eines Rapid-Prototyping-Verfahrens gebildet ist. Vorzugsweise sind auch die Markierungen mittels des Rapid-Prototyping-Verfahrens eingebracht. Dies ist einerseits eine kostengünstige Art der Herstellung des Manipulators, insbesondere wenn dieser als Single-Use-Produkt verwendet wird. Diese Ausführungsform hat auch den Vorteil, dass der Manipulator vollständig sterilisierbar ist, da keine Elektronik in dem Manipulator vorgesehen ist. Ferner ist es auch möglich auf diese Weise jeden Manipulator patientenindividuell anzupassen. So können Teile einer Kinematik des Manipulators bei Kindern kleiner sein als bei Erwachsenen. In diesem Fall ist eine Planungssoftware für den chirurgischen Eingriff bevorzugt, die dann aus den anatomischen Strukturen und den Planungsdaten des Chirurgen automatisch die optimale Kinematik berechnet und ausdruckt.

Gemäß einer weiteren bevorzugten Ausführungsform ist der Manipulator vollständig aus einem Kunststoff gebildet. Bevorzugt wird PA12 oder ein vergleichbarer Kunststoff eingesetzt. Durch das Ausbilden des Manipulators aus Kunststoff ist dieser durchleuchtbar für Strahlung, insbesondere Röntgenstrahlung und auch verwendbar in einem Computertomographen oder Kernspintomographen. Hierdurch ist der Einsatzbereich des Manipulators weiter vergrößert. Da der Manipulator originär keinen Antrieb aufweist, ist es möglich, diesen so zu bilden, dass er in einem Kernspintomographen eingesetzt werden kann.

Weiterhin ist bevorzugt, dass die erste Lenkeranordnung einen ersten und einen zweiten Hebel aufweist, die relativ zueinander und relativ zum Rahmen um Schwenkachsen schwenkbar gelagert sind und die Schwenkachsen parallel zueinander verlaufen, und die zweite Lenkeranordnung einen dritten und einen vierten Hebel aufweist, die relativ zueinander und relativ zum Rahmen um Schwenkachsen schwenkbar gelagert sind und die Schwenkachsen parallel zueinander und zu den Schwenkachsen der ersten Lenkeranordnung verlaufen. Hierdurch ist insbesondere der Bauraum des Manipulators verkleinert und eine kompakte Konstruktion erreicht.

Die eingangs genannte Aufgabe wird im zweiten Aspekt der Erfindung durch eine handgehaltene Antriebseinheit zur Verwendung mit einem passiven chirurgischen Manipulator nach einer der vorstehend beschriebenen bevorzugten Ausführungsformen eines passiven chirurgischen Manipulators gelöst, mit einem Gehäuse, in dem ein Antriebsmotor in einer Steuereinheit mit einem Prozessor und Softwaremitteln angeordnet ist, und einer Schnittstelle zum temporären und sequenziellen Koppeln mit einer korrespondierenden Schnittstelle des Manipulators und zum Übertragen einer Stellbewegung auf den Manipulator, wobei die Steuereinheit dazu eingerichtet ist, den Antriebsmotor derart zu steuern, dass ein Stellbewegung übertragen wird, die dazu geeignet ist, die ersten und zweiten Gelenkpunkte zu bewegen und in einer vorbestimmten Sollposition zu positionieren. Es soll verstanden werden, dass der Manipulator gemäß dem ersten Aspekt der Erfindung und die Antriebseinheit gemäß dem zweiten Aspekt der Erfindung gleiche und ähnliche bevorzugte Weiterbildungen aufweisen, wie sie insbesondere in den Unteransprüchen niedergelegt sind. Insofern wird vollumfänglich auf die obige Beschreibung des Manipulators gemäß dem ersten Aspekt der Erfindung verwiesen.

Die handgehaltene Antriebseinheit kann nach ihrer Art her wie ein handgehaltenes Schleifgerät, oder eine handgehaltene elektrische Zahnbürste ausgebildet sein. Sie ist vorzugsweise im Wesentlichen stabförmig und weist vorzugsweise an einem axialen Ende die Schnittstelle zum temporären und sequenziellen Koppeln mit der korrespondierenden Schnittstelle des Manipulators auf.

Gemäß einer ersten bevorzugten Ausführungsform der Antriebseinheit weist die Schnittstelle der Antriebseinheit eine Abtriebswelle auf. Die Abtriebswelle weist vorzugsweise einen elektronischen Kontakt zur Übertragung von Signalen auf. Auf diese Weise können von der Antriebseinheit auch elektronische Signale an den Manipulator übertragen werden, oder von dem Manipulator erfasst werden. Beispielsweise, für den Fall, dass der Manipulator Sensoren aufweist zur Erfassung von Kräften oder Drehstellungen einzelner Elemente, können von diesen Sensoren entsprechend Signale über die Schnittstelle auf die Antriebseinheit übertragen werden.

Ferner ist bevorzugt, dass die Abtriebswelle Kupplungsmittel zum Kuppeln mit den Schnittstellen des Manipulators aufweist. Diese Kupplungsmittel können formschlüssig oder kraftschlüssig, insbesondere magnetisch wirken, wie dies bereits oben mit Bezug auf den Manipulator beschrieben wurde.

Hierbei ist besonders bevorzugt, dass die Abtriebswelle für jede Schnittstelle des Manipulators ein separates Kupplungsmittel aufweist. Dies ist insbesondere dann bevorzugt, wenn die Schnittstellen des Manipulators in einer gemeinsamen Kupplungsausnehmung angeordnet sind. Hier wird insbesondere ein Schlüsselprinzip umgesetzt. Beispielsweise weist die Abtriebswelle in der Antriebseinheit vier Zahnräder oder dergleichen auf, die axial versetzt sind und von der Spitze aus zur Antriebseinheit hin im Durchmesser zunehmen. Entsprechende Scheiben mit Ausnehmungen weist der Manipulator auf, sodass auf diese Weise die Antriebseinheit mit dem Manipulator gekoppelt werden kann.

Gemäß einer weiteren bevorzugten Weiterbildung ist vorgesehen, dass die Steuereinheit dazu ausgebildet ist, eine vorbestimmte Sollposition des ersten und zweiten Gelenkpunkts zu empfangen. Diese Sollposition wird vorzugsweise von einem OP-Navigationssystem oder dergleichen bereitgestellt. Die Steuereinheit der Antriebseinheit kennt die Kinematik des Manipulators und entsprechende Daten sind hierzu in der Steuereinheit der Antriebseinheit hinterlegt. Basierend hierauf und basierend auf der gewünschten Sollposition und der Ist-Position des Manipulators wird die erforderliche Stellbewegung von der Steuereinheit der Antriebseinheit ermittelt.

Die Antriebseinheit ist dann vorzugsweise dazu ausgebildet, basierend auf der vorbestimmten Sollposition des ersten und zweiten Gelenkpunkts eine an jeder Schnittstelle erforderliche Stellbewegung zum Erreichen der Sollposition zu ermitteln.

Weiter ist bevorzugt, dass die Antriebseinheit ein Anzeigepaneel aufweist, wobei die Steuereinheit dazu eingerichtet ist, ein Erreichen der Sollstellung mittels des Anzeigepaneels anzuzeigen. Die Antriebseinheit überträgt die Stellbewegung auf den Manipulator. Das heißt, der Abtrieb der Antriebseinheit hört auf sich zu bewegen, wenn die Sollposition erreicht ist. Dennoch ist es sinnvoll, das Erreichen der Sollposition anzuzeigen. Je nach Umfang und Geschwindigkeit der Bewegung ist die Dauer der Übertragung der Bewegung auf den Manipulator unterschiedlich lang und daher ist es bevorzugt, den Bediener darüber zu informieren, dass die Sollposition erreicht wurde. Der Bediener kann dann die Antriebseinheit von dem Manipulator entfernen und an einer weiteren Schnittstelle einsetzen.

Auch ist bevorzugt, dass die Antriebseinheit eine Aufnahme für einen Navigationstracker aufweist. Hierdurch kann ein Navigationssystem registrieren, dass ein Bediener die Antriebseinheit in das OP-Feld eingebracht hat und das OP-Navigationssystem kann basierend hierauf ein oder mehrere Einrichtungen veranlassen, ein oder mehrere Aktionen auszuführen, oder zu unterbinden.

Weiterhin ist bevorzugt, dass die Antriebseinheit wenigstens eine Infrarotstrahlungsquelle, insbesondere IR-LED zum Senden von Signalen an ein chirurgisches Navigationssystem aufweist. Solche Informationen können insbesondere das Erreichen der Sollposition, oder auch erfasste Daten des Manipulators sein, wie insbesondere Kräfte an den Gelenken. Auch kann die Antriebseinheit die Sollposition selbst an das OP-Navigationssystem übertragen, sodass das OP-Navigationssystem die Position des Instruments im OP-Feld kennt. Für diesen Fall ist eine separate Ausrüstung des Instruments mit Navigationstrackern nicht erforderlich.

Gemäß einer weiteren Ausgestaltung der Antriebseinheit ist vorgesehen, dass diese wenigstens einen Sensor zum Ermitteln eines an der Schnittstelle wirkenden mechanischen Widerstands aufweist, wobei der Sensor mit der Steuereinheit verbunden ist und die Steuereinheit dazu eingerichtet ist, die erfassten Daten zu verarbeiten und/oder an einem Peripheriegerät bereitzustellen. So ist erfassbar, ob das Instrument, welches an dem Manipulator aufgenommen ist, bei der Bewegung von der Ist-Position in die Soll-Position mit Widerständen in Kontakt kommt, und ob etwa hierdurch ein Patient verletzt werden könnte. Hierdurch ist die Sicherheit weiter verbessert. Es kann beispielsweise vorgesehen sein, dass die Steuereinheit und/oder das Peripheriegerät bestimmt, dass ein mechanischer Widerstand einen vorbestimmten Schwellwert überschreitet, und dass das weitere Verbringen des Manipulators in die Soll-Stellung verhindert wird.

Vorzugsweise umfasst das Verarbeiten: Ermitteln von auf den ersten und zweiten Gelenkpunkten des Manipulators wirkenden Kräften. Insbesondere werden Kräfte erfasst und ermittelt, die auf einen Tool Center Point wirken.

Gemäß einem dritten Aspekt der Erfindung wird die eingangs genannte Aufgabe durch ein chirurgisches System gelöst, umfassend wenigstens einen Manipulator nach einer der vorstehend beschriebenen bevorzugen Ausführungsformen eines passiven chirurgischen Manipulators gemäß dem ersten Aspekt der Erfindung und einer Antriebseinheit gemäß einer der vorstehend beschriebenen bevorzugten Ausführungsformen einer handgehaltenen Antriebseinheit gemäß dem zweiten Aspekt der Erfindung.

In einem vierten Aspekt löst die Erfindung die eingangs genannte Aufgabe durch ein Verfahren zum Einstellen einer Soll-Position von zwei Gelenkpunkten eines passiven chirurgischen Manipulators, insbesondere einem Manipulator nach einer der vorstehend beschriebenen bevorzugten Ausführungsformen eines passiven chirurgischen Manipulators gemäß dem ersten Aspekt der Erfindung, mittels einer handgehaltenen Antriebseinheit, insbesondere einer Antriebseinheit nach einem der vorstehend beschriebenen bevorzugten Ausführungsform einer handgehaltenen Antriebseinheit gemäß dem zweiten Aspekt der Erfindung, wobei das Verfahren die Schritte aufweist: Ermitteln einer Ist-Position der zwei Gelenkpunkte; Ermitteln einer Soll-Position der zwei Gelenkpunkte; Ermitteln von Stellbewegungen für die Lenkeranordnungen des Manipulators und Übertragen der Stellbewegung mittels der handgehaltenen Antriebseinheit an dafür vorgesehenen Schnittstellen des Manipulators durch temporäres und sequenzielles Kuppeln der handgehaltenen Antriebseinheit mit den entsprechenden Schnittstellen des Manipulators. Es soll verstanden werden, dass der Manipulator gemäß dem ersten Aspekt der Erfindung, die Antriebseinheit gemäß dem zweiten Aspekt der Erfindung sowie das System gemäß dem dritten Aspekt der Erfindung und das Verfahren gemäß dem vierten Aspekt der Erfindung gleiche und ähnliche bevorzugte Ausgestaltungen aufweisen, wie sie insbesondere in den Unteransprüchen niedergelegt sind. Insofern wird vollumfänglich auf die obige Beschreibung zu dem ersten, zweiten und dritten Aspekt der Erfindung Bezug genommen.

Bei dem Verfahren gemäß dem vierten Aspekt der Erfindung umfasst das Übertragen der Stellbewegung vorzugsweise: Kuppeln der handgehaltenen Antriebseinheit mit einer ersten Schnittstelle des Manipulators; Betätigen der Antriebseinheit und Übertragen einer ersten Stellbewegung auf die erste Schnittstelle zum Bewegen eines ersten Lenkers, insbesondere Hebels, des Manipulators; Entkuppeln der handgehaltenen Antriebseinheit von der ersten Schnittstelle; Kuppeln der handgehaltenen Antriebseinheit mit einer zweiten Schnittstelle des Manipulators; Betätigen der Antriebseinheit und Übertragen einer zweiten Stellbewegung auf die zweite Schnittstelle zum Bewegen eines zweiten Lenkers, insbesondere Hebels, des Manipulators; und Entkuppeln der handgehaltenen Antriebseinheit von der zweiten Schnittstelle. Die Schritte des Übertragens der Stellbewegung werden vorzugsweise in dieser Reihenfolge ausgeführt.

Nachstehend wird die Erfindung anhand von Ausführungsbeispielen näher erläutert. Dabei zeigen:
- Figur 1: eine perspektivische Ansicht eines passiven chirurgischen Manipulators, der an einem robotischen Haltearm befestigt ist;
- Figur 2: eine schematische Draufsicht auf den Manipulator aus Figur 1;
- Figur 3: eine schematische Teilansicht einer Schnittstelle des Manipulators aus Figur 2;
- Figur 4: eine erste Variante der Anordnung von Schnittstellen in einer schematischen Darstellung;
- Figur 5: eine zweite Variante der Anordnung der Schnittstellen in einer schematischen Ansicht;
- Figur 6: einen schematischen Schnitt durch eine handgehaltene Antriebseinheit gemäß einem ersten Ausführungsbeispiel;
- Figur 7: die handgehaltene Antriebseinheit gemäß Figur 6, während sie mit einer Schnittstelle des Manipulators gekoppelt ist;
- Figur 8: eine schematische Seitenansicht einer zweiten Ausführungsform der handgehaltenen Antriebseinheit;
- Figur 9: eine schematische Draufsicht auf eine Kinematik eines Manipulators gemäß einem zweiten Ausführungsbeispiels;
- Figur 10: eine schematische Draufsicht eines Manipulators gemäß einem dritten Ausführungsbeispiel; und
- Figur 11: eine schematische Darstellung eines Verfahrens gemäß der Erfindung.

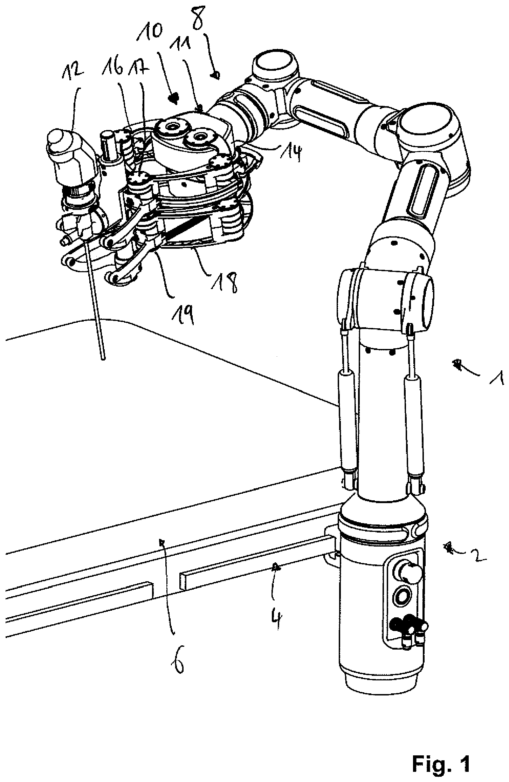

Gemäß Figur 1 ist zunächst ein robotischer Haltearm 1 gezeigt, wie er grundsätzlich aus DE 10 2014 016 842 A1, DE 10 2014 016 823 A1 und DE 10 2015 104 819 A1 bekannt ist. Deren Offenbarungsgehalt wird vollumfänglich hierin mit einbezogen. Der Haltearm 1 weist ein proximales Ende 2 auf, mit dem diese an einer Schiene 4 eines OP-Tisches 6 befestigt ist. An dem distalen Ende 8 des Haltearms 1 ist ein passiver chirurgischer Manipulator 10 aufgenommen. Es soll verstanden werden, dass der passive chirurgischer Manipulator 10 nicht nur an dem Haltearm 1 aufgenommen werden kann, sondern auch an anderen Haltesystemen, wie insbesondere Stativen oder Robotern. Zum Befestigen des Manipulators 10 an dem Haltearm 1 weist dieser einen Befestigungsabschnitt 11 auf. Der Manipulator 10 trägt ferner ein chirurgisches Instrument 12, das in diesem Ausführungsbeispiel als Endoskop ausgebildet ist.

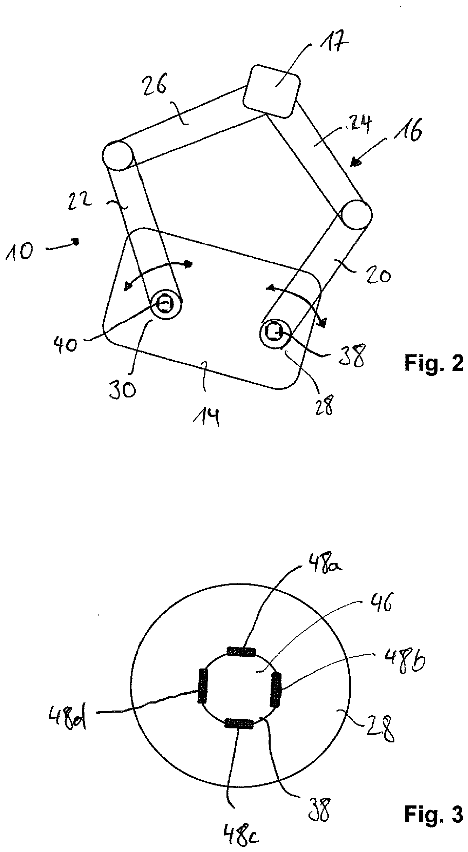

Der passive chirurgische Manipulator 10 weist einen Rahmen 14 auf (vgl. Fig. 2) an dem eine erste Lenkeranordnung 16 und eine zweite Lenkeranordnung 18 (vgl. Fig. 1) gelenkig angeordnet sind. In Figur 2 ist nur die erste Lenkeranordnung 16 zu sehen, die zweite Lenkeranordnung 18 ist allerdings identisch ausgebildet (vgl. Fig. 1) und mit Bezug auf Figur 2 in der Zeichenebene hinter der ersten Lenkeranordnung 16 angeordnet. Die erste Lenkeranordnung 16 weist einen ersten Gelenkpunkt 17 auf und die zweite Lenkeranordnung einen zweiten Gelenkpunkt 19. An den beiden Gelenkpunkten 17, 19 ist (in Figur 1 über eine weitere Halterung) das Instrument 12 aufgenommen. Durch entsprechende verschiedene Positionierungen der ersten und zweiten Gelenkpunkte 17, 19 ist eine Verkippung des Instruments 12 möglich.

Die Aussagen zur ersten Lenkeranordnung 16 sollen im Folgenden auch für die zweite Lenkeranordnung 18 entsprechend gelten, auch wenn dies nicht explizit erwähnt ist.

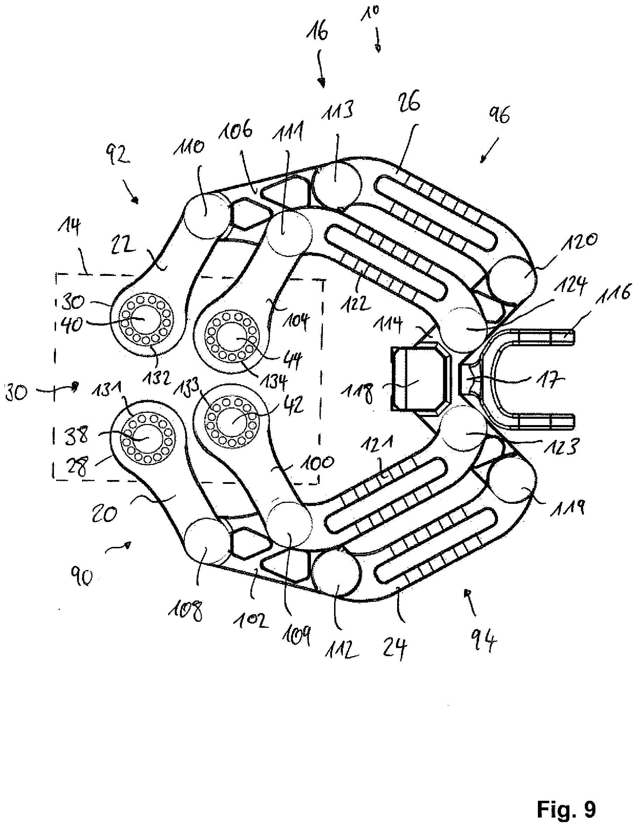

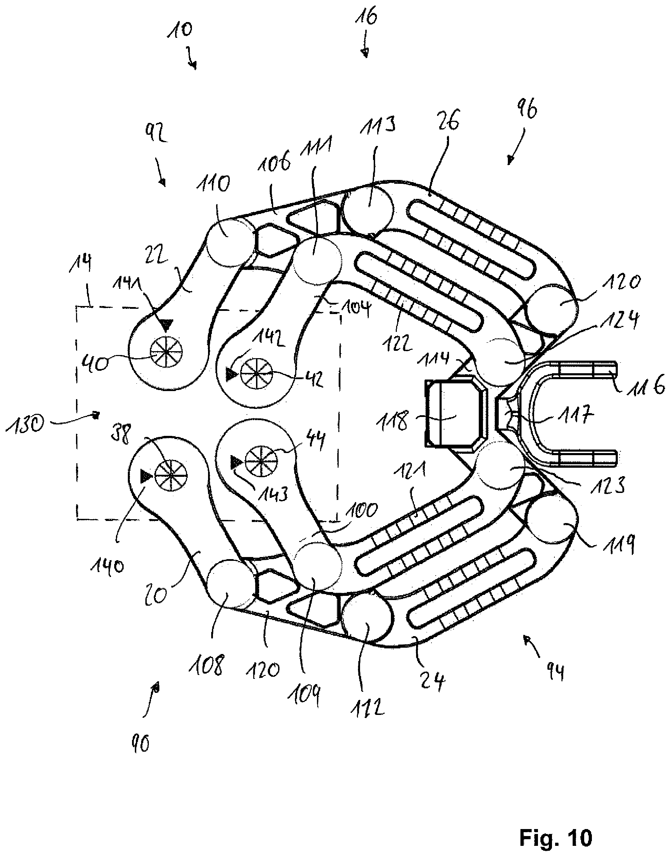

Die ersten Lenkeranordnung 16 umfasst als Lenker einen ersten Hebel 20 und einen zweiten Hebel 22 sowie einen ersten Stab 24 und einen zweiten Stab 26. Es soll verstanden werden, dass auch andere Konfigurationen denkbar sind, wie dies insbesondere mit Bezug auf die Figuren 9 und 10 später erläutert werden wird.

Auch ist es denkbar die vorliegende Erfindung auf eine sogenannte Vier-Finger-Kinematik anzuwenden, bei der vier Finger es Manipulators parallel zueinander und translatorisch verschiebbar angeordnet sind. Die vier Finger sind endseitig mit einer Übertragungskinematik mit einem Instrument gekoppelt. Durch entsprechende lineare Verschiebung jedes Fingers ist so das Instrument positionierbar. In diesem Sinne bilden die Finger Lenker im Sinne der Erfindung. Das Gleiche gilt auch für Drei-Finger-Kinematiken oder Kinematiken mit fünf oder mehr Fingern.

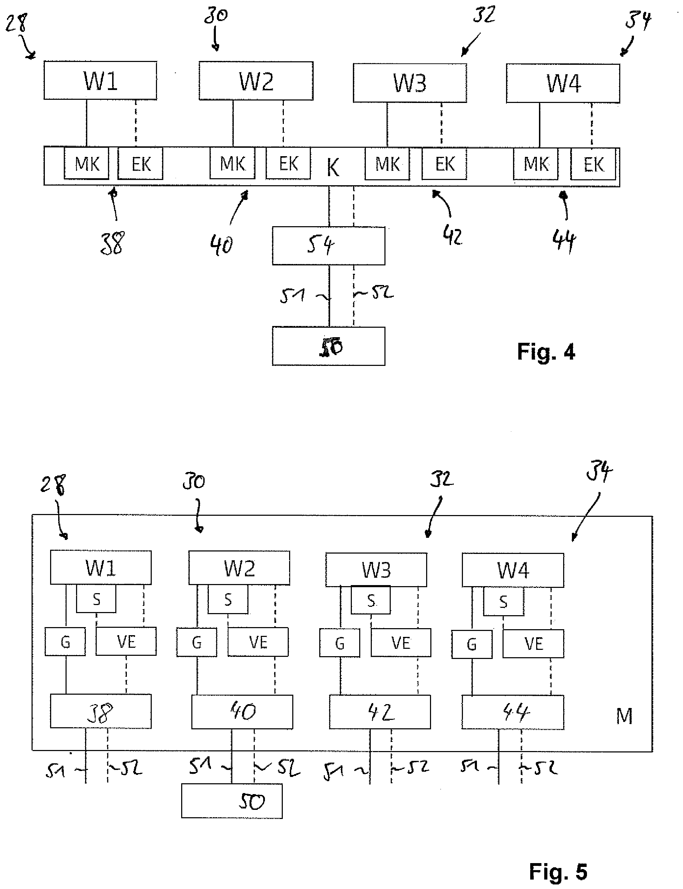

Die erste Lenkeranordnung 16 ist an ersten und zweiten Stelleinrichtungen 28, 30 mit dem Rahmen 14 verbunden. Ebenso ist die zweite Lenkeranordnung 18 an dritten und vierten Stelleinrichtungen 32, 34 (in Fig. 2 nicht zu sehen, vgl. Fig. 5) mit dem Rahmen 14 verbunden. Über die Stelleinrichtungen 28, 30, 32, 34 sind die ersten und zweiten Hebel 20, 22 sowie auch dritte und vierte Hebel 24, 26 der zweiten Lenkeranordnung 18 (vgl. Fig. 5) stellbar, wie durch die Pfeile in Figur 2 angezeigt.

Erfindungsgemäß weist die erste Stelleinrichtung 28 eine erste Schnittstelle 38 auf, die zweite Stelleinrichtung eine zweite Schnittstelle 40, die dritte Stelleinrichtung 32 eine dritte Schnittstelle 42 und die vierte Stelleinrichtung 34 eine vierte Schnittstelle 44. Es ist allerdings auch möglich, dass weitere Schnittstellen, wie insbesondere fünfte, sechste, etc. vorgesehen sind. Dies ist insbesondere abhängig von der in der konkreten Ausführung gewählten Kinematik.

Figur 3 zeigt eine vergrößerte Darstellung der Stelleinrichtung 38. Die Stelleinrichtung 38 ist als drehbare Scheibe mit einer Öffnung 46, die die Schnittstelle 38 aufweist, ausgebildet. Radial innerhalb der Öffnung 46 sind elektrische Kontakte 48a, 48b, 48c, 48d angeordnet, um Daten zu empfangen und zu übertragen. An der Stelleinrichtung 38 ist dann der Hebel 20 gekoppelt. Die Stelleinrichtung 38 und Hebel 20 können auch einteilig ausgebildet sein, insbesondere nicht zerstörungsfrei lösbar.

Die Figuren 4 und 5 illustrieren zwei verschiedene Ausführungsbeispiele der Schnittstellen 38, 40, 42, 44.

Zunächst Bezug nehmend auf Figur 4 ist eine Variante beschrieben, bei der die Schnittstellen 38, 40, 42, 44 nicht räumlich voneinander getrennt sind. In Figur 4 sind diese zwar nebeneinander dargestellt, es soll aber verstanden werden, dass sie einheitlich in einer gemeinsamen Kupplungsausnehmung 54 angeordnet sind. Die handgehaltene Antriebseinheit 50 ist in diesem Fall nur in die gemeinsame Kupplungsausnehmung 54 einzusetzen und kommt gleichzeitig mit allen vier Schnittstellen 38, 40, 42, 44 in Kontakt. Diese sind innerhalb der gemeinsamen Kupplungsausnehmung 54 separiert und können separat angesteuert werden. Die separaten Schnittstellen 38, 40, 42, 44 weisen jeweils eine mechanische Kupplung MK sowie eine elektronische Kupplung EK mit einer integrierten Verarbeitungseinheit auf. Insofern weist die Kupplungsausnehmung 54 eine mechanische Kupplung 51 mit der handgehaltenen Antriebseinheit 50 auf sowie eine elektrische Verbindung 52, die über die gestrichelte Linie dargestellt ist. Auf gleiche Weise sind auch die mechanischen Kopplungen MK über eine mechanische Kupplung mit entsprechenden Antriebswellen W1, W2, W3, W4 für die ersten, zweiten, dritten und vierten Stelleinrichtungen 28, 30, 32, 34 gekoppelt, sowie darüber hinaus mit einer über eine elektrische Leitung 52 mit den elektronischen Kupplungen EK.

Bei dem zweiten Ausführungsbeispiel der Figur 5 sind die Schnittstellen 38, 40, 42, 44 räumlich voneinander getrennt. Je eine Schnittstelle 38, 40, 42, 44 ist in einer Drehachse der Hebel 20, 22, 24, 26, das heißt auch in der Drehachse der Stelleinrichtungen 28, 30, 32, 34 angeordnet. Insbesondere ist die jeweilige Öffnung 46 der Schnittstellen 38, 40, 42, 44 koaxial zur Drehachse der Hebel 20, 22, 24, 26 angeordnet. In der in Figur 5 gezeigten Stellung ist eine handgehaltene Antriebseinheit 50 mit der zweiten Schnittstelle 40 gekoppelt. Mittels der durchgezogenen Linie 51 ist die mechanische Verbindung angezeigt und mittels der gestrichelten Linie 52 die elektrische Verbindung. Die Schnittstelle 40 ist ihrerseits über eine Getriebestufe G sowie eine Verarbeitungseinheit VE mit einer entsprechenden Antriebswelle W2 für die zweite Stelleinrichtung 30 gekoppelt. Die Verarbeitungseinheit VE ist über die elektrische Leitung mit einem Sensor S gekoppelt, der seinerseits eine Kraft misst, die auf die zweite Welle wirkt.

Wie sich aus Figur 5 ergibt, ist jeder der Schnittstellen 38, 40, 42, 44 mit entsprechenden Leitungen 51, 52 ausgestattet sowie auch mit entsprechenden Getriebestufen G, Verarbeitungseinheiten VE, Sensoren S und Wellen W1, W2, W3 und W4.

Die Ausführungsformen der Figuren 4 und 5 können auch kombiniert werden. So ist es insbesondere denkbar, bei der Variante gemäß Figur 4 die Sensoren der Variante gemäß Figur 5 einzusetzen. Auch ist es bevorzugt, dass bei der Variante gemäß Figur 4 separate Verarbeitungseinheiten VE gemäß Figur 5 eingesetzt werden.

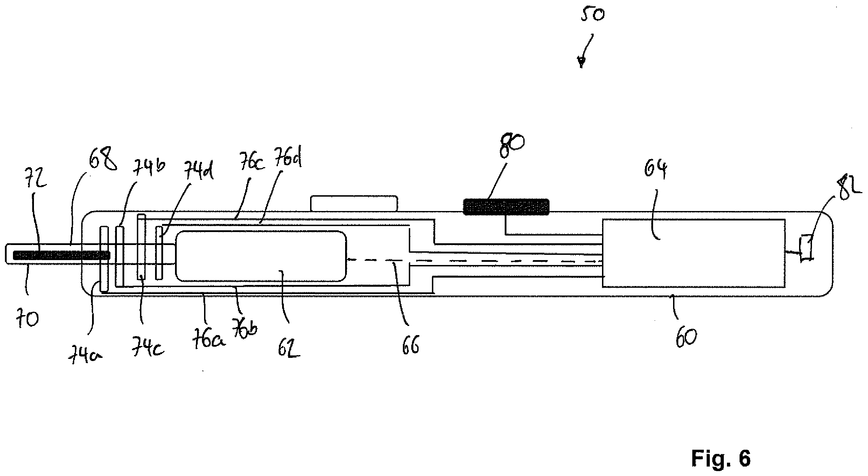

Figur 6 illustriert eine handgehaltene Antriebseinheit 50, wie sie mit der Ausführungsform gemäß Figur 5 verwendet werden kann. Das heißt, die Antriebseinheit 50 gemäß Figur 6 ist dazu vorgesehen, mit räumlich voneinander getrennten, separaten Schnittstellen 38, 40, 42, 44 in Kontakt zu kommen und nicht mit Schnittstellen, die in einer gemeinsamen Kupplungsausnehmung 54 angeordnet sind.

Die handgehaltene Antriebseinheit 50 weist ein Gehäuse 60 auf, das in diesem Ausführungsbeispiel im Wesentlichen stabförmig ist und in seiner Konfiguration einer elektrischen Zahnbürste oder einem Handschleifer ähnelt. Im Inneren des Gehäuses 60 ist ein Antriebsmotor 62 vorgesehen, der als Elektromotor ausgebildet ist. Der Motor 62 ist insbesondere als Stellmotor ausgebildet. Der Motor 62 ist verbunden mit einer Steuereinheit 64, von der Steuereinheit 64 hält der Motor 62 über eine hier gestrichelt dargestellte Leitung 66 elektrische Energie und Stellsignale, das heißt insbesondere Signale für eine Bewegung seiner Abtriebswelle 68. Die Abtriebswelle 68 der Antriebseinheit 50 bildet eine Schnittstelle 70 zum temporären und sequenziellen Kuppeln mit einer korrespondierenden Schnittstelle 38, 40, 42, 44 des Manipulators und zum Übertragen einer Stellbewegung auf den Manipulator 10. Dazu weist die Abtriebswelle 68 gemäß diesem Ausführungsbeispiel einen elektrischen Kontakt 72 auf, zum Kontaktieren der entsprechenden elektrischen Kontakte 48a, 48b, 48c, 48d, die mit der Verarbeitungseinheit VE beziehungsweise der elektronischen Kupplung EK verbunden sind. Zum Abgreifen und Übertragen von Daten und Signalen auf den elektrischen Kontakt 72 sind vier Schleifringe 74a, 74b, 74c, 74d vorgesehen, die ihrerseits über Signalleitung 76a, 76b, 76c, 76d mit der Steuereinheit 64 gekoppelt sind.

Zum Kuppel der Abtriebswelle 68 mit den Schnittstellen 38, 40, 42, 44 ist diese abschnittsweise vollständig magnetisch ausgebildet, und die Schnittstellen 38, 40, 42, 44 weisen korrespondierende, insbesondere ferromagnetische Kupplungselemente auf, sodass eine Drehbewegung von der Abtriebswelle 68 kraftschlüssig auf die ersten und zweiten Lenkeranordnungen 16, 18 übertragbar ist.

Schließlich weist die Antriebseinheit 50 ein Anzeige-Bedienpaneel 80 auf, über das die Antriebseinheit 50 betätigbar ist.

Die Steuereinheit 64 ist dazu ausgebildet, über eine Drahtlosschnittstelle 82 eine vorbestimmte Soll-Position für die ersten und zweiten Gelenkpunkte 17, 19 zu empfangen und basierend auf dieser empfangenen vorbestimmten Soll-Position eine an jeder Schnittstelle 38, 40, 42, 44 erforderliche Stellbewegung zum Erreichen der Soll-Position zu ermitteln. Dazu ist die Konfiguration der ersten und zweiten Lenkereinheiten 16, 18 in einer Speichereinheit in der Steuereinheit 64 hinterlegt und die Steuereinheit 64 ermittelt mittels entsprechender Softwaremittel basierend auf der aktuellen Ist-Position des ersten und zweiten Gelenkspunktes 17, 19 und der Soll-Position der Gelenkpunkte 17, 19 die erforderliche Stellbewegung an jeder Schnittstelle 38, 40, 42, 44. Die erforderliche Stellbewegung ist in diesem Ausführungsbeispiel dann durch eine entsprechende Drehung der Abtriebswelle 68 der Antriebseinheit 50 verkörpert.

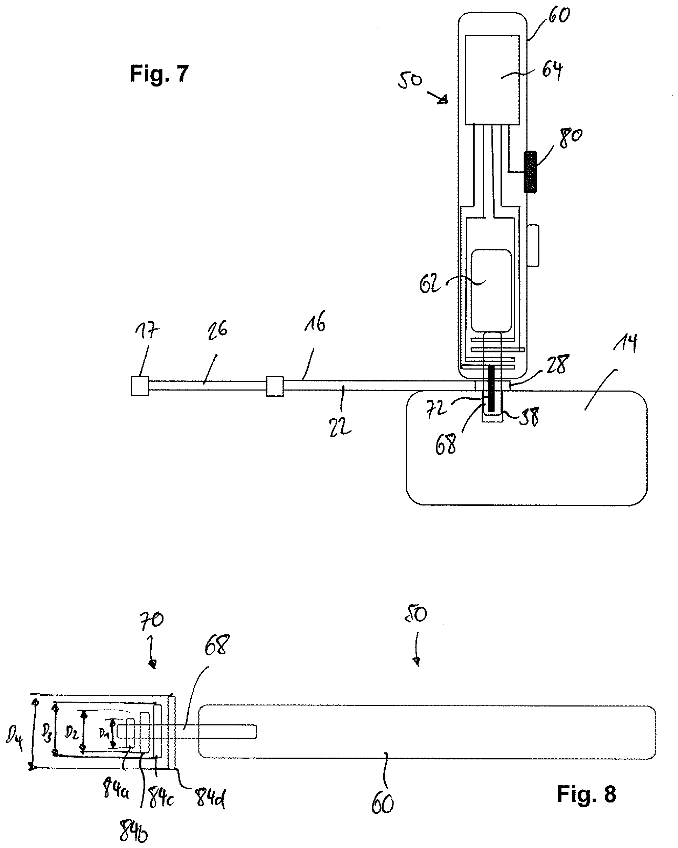

Zum Übertragen der Stellbewegung wird in diesem Ausführungsbeispiel die handgehaltene Antriebseinheit 50 zunächst mit einer Schnittstelle, in diesem Ausführungsbeispiel der ersten Schnittstelle 38, gekoppelt (Fig. 7). Hierzu wird die Abtriebswelle 68 in die Schnittstelle 38 gesteckt. Dass der Bediener diese Operation durchführen soll wird dem Bediener über das Anzeige-Bedienpaneel 80 angezeigt. Auf dem Anzeige-Bedienpaneel 80 kann beispielsweise angezeigt werden, dass der Bediener die Antriebseinheit 50 mit der ersten Schnittstelle 38 koppeln soll. Ist dies erfolgt, betätigt die Steuereinheit 64 den Motor 62 und der Motor treibt die Abtriebswelle 68 solange an, bis der entsprechende Drehwinkel, der durch die Steuereinheit 64 vorgegeben ist, erreicht wurde. Anschließend schaltet die Steuereinheit 64 den Antrieb 62 wieder aus. Hierdurch wird der erste Hebel 22 verschwenkt und über den ersten Stab 26 der Gelenkpunkt 17 bewegt.

Nachdem nun die Stellbewegung auf die erste Schnittstelle 38 übertragen wurde, kann ein Bediener die Antriebseinheit 50 wieder von der Schnittstelle 38 entkoppeln und auf dem Anzeige-Bedienpaneel 80 wird ein entsprechendes Signal ausgegeben. Anschließend erscheint auf dem Anzeige-Bedienpaneel 80 ein weiteres Signal, das dem Bediener anzeigt, mit welcher der Schnittstellen 38, 40, 42, 44 die Antriebseinheit 50 als Nächstes zu koppeln ist.

In der oben beschriebenen Variante der Figur 4, bei der die Schnittstellen 38, 40, 42, 44 in einer gemeinsamen Kupplungsausnehmung 54 ausgebildet sind, ist die Antriebseinheit 50 nur mit dieser einen Kupplungsausnehmung 54 zu koppeln. Dazu weist die Antriebseinheit 50 eine speziell dafür vorgesehene Schnittstelle 70 auf, die in Figur 8 dargestellt ist. Grundsätzlich ist die Antriebseinheit 50 gemäß dem zweiten Ausführungsbeispiel (Fig. 8) identisch zu der Antriebseinheit 50 gemäß dem ersten Ausführungsbeispiel (Figuren 6, 7) ausgebildet. Der Unterschied besteht im Wesentlichen in der Schnittstelle 70. Die Schnittstelle 70 ist so ausgebildet, dass sie gleichzeitig mit allen vier Schnittstellen 38, 40, 42, 44, die gemeinsam in der gemeinsamen Kupplungsausnehmung 54 angeordnet sind, kuppeln kann. Dazu sind an der Abtriebswelle 68 vier Abtriebsscheiben 84a, 84b, 84c, 84d angeordnet, deren äußerer Durchmesser D1, D2, D3, D4 vom distalen Ende der Abtriebswelle 68 zum proximalen Ende, das heißt zum Gehäuse 60 hin, zunimmt. So kann jede der Abtriebsscheiben 84a, 84b, 84c, 84d mit einem entsprechenden Kupplungselement der ersten, zweiten, dritten und vierten Schnittstelle 38, 40, 42, 44 in Kontakt kommen und so die Stellbewegung übertragen. Vorzugsweise sind die einzelnen Abtriebsscheiben 84a, 84b, 84c, 84d separat antreibbar, beispielsweise über mehrere ineinander geschachtelte Hohlwellen in der Abtriebswelle 68. Die Abtriebsscheiben 84a, 84b, 84c, 84d können beispielsweise als Zahnräder ausgebildet sein. In der Kupplungsausnehmung 54 können entsprechende Scheiben mit zentralen Durchlasslöchern ausgebildet sein, oder axial versetzt zur Abtriebswelle 68 separate Kupplungselemente, die dann um entsprechende Rotationsachsen rotieren können, die parallel versetzt zur Abtriebswelle 68 sind.

Figur 9 illustriert nun ein zweites Ausführungsbeispiel des Manipulators 10, wobei in Figur 9 nur die Kinematik des Manipulators 10, nämlich die erste Lenkeranordnung 16 dargestellt ist. Gleiche und ähnliche Elemente sind mit gleichen Bezugszeichen versehen und insofern wird vollumfänglich auf die obige Beschreibung Bezug genommen.

Die Kinematik der Lenkeranordnung 16 ist in diesem Ausführungsbeispiel (Fig. 9) als doppelte Kinematik ausgebildet und weist insofern ein erstes, zweites, drittes und viertes Parallelogramm 90, 92, 94, 96 auf. Das erste Parallelogramm 90 wird durch den ersten Hebel 20 aufgespannt und das zweite Parallelogramm 92 durch den zweiten Hebel 22. Genauer gesagt wird das erste Parallelogramm 90 durch den ersten Hebel 20, einen fünften Hebel 100 und eine erste Lenkerplatte 102 sowie den Rahmen 14 (in Fig. 9 nur schematisch gezeigt) gebildet. Das zweite Parallelogramm 92 wird in entsprechender Weise durch den zweiten Hebel 22, einen sechsten Hebel 104, eine zweite Lenkerplatte 106 sowie den Rahmen 14 gebildet. Die Lenker 20, 22, 100, 104 sind über Gelenkpunkte 108, 109, 110, 111 mit der jeweiligen Lenkerplatte 102, 106 gelenkig verbunden.

An die Lenkerplatten 102, 106 schließen sich dann die ersten und zweiten Stäbe 24, 26 an, die ihrerseits über Gelenkpunkte 112, 113 mit der jeweiligen Lenkerplatte 102, 106 verbunden sind. Am anderen Ende sind die ersten und zweiten Stäbe 24, 26 mit einer Klammer 114 verbunden, die den Gelenkpunkt 17 für das Instrument bildet. Über den Gelenkpunkt 17 ist in diesem Ausführungsbeispiel ein weiterer U-Halter 116 an der Klammer 114 angeordnet, der dazu vorgesehen ist, das Instrument aufzunehmen. Genauer gesagt ist der U-Halter 116 mit einem Linearantrieb 118 gekoppelt, der den U-Halter 116 mit Bezug auf Figur 9 in Zeichenebene linear bewegen kann.

Die ersten und zweiten Stäbe 24, 26 sind über Gelenkpunkte 119, 120 mit der Klammer 114 verbunden. Zum Aufspannen der dritten und vierten Parallelogramme 94, 96 sind ferner fünfte und sechste Stäbe 121, 122 vorgesehen, die einerseits am Gelenkpunkt 109, 111 mit den Lenkerplatten 102, 106 verbunden sind sowie am anderen Ende über Gelenkpunkte 123, 124 mit der Klammer 114. Durch diese besondere Anordnung der Parallelogramme 90, 92, 94, 96 wird eine besondere Stabilität erreicht. Zum Bewegen des ersten Gelenkpunktes 17 reicht es aus, zwei der Hebel, insbesondere die ersten und zweiten Hebel 20, 22 zu verstellen. Die fünften und sechsten Hebel 100, 104 können passiv mitlaufen.

Insofern sind die ersten und zweiten Hebel 20, 22, wie mit Bezug auf die vorherigen Ausführungsbeispiele beschrieben wurde, mit ersten und zweiten Stelleinrichtungen 28, 30 gekoppelt, die ersten und zweiten Schnittstellen 38, 40 aufweisen. Das heißt, zum Verstellen der ersten Lenkeranordnung gemäß diesem Ausführungsbeispiel (Fig. 9) kann die handgehaltene Antriebseinheit 50 gemäß dem ersten Ausführungsbeispiel (Fig. 6) mit der ersten Schnittstelle 38 und/oder der zweiten Schnittstelle 40 gekoppelt werden, um die ersten und zweiten Hebel 20, 22 anzutreiben, während die fünften und sechsten Hebel 100, 104 passiv mitlaufen.

In Figur 9 ist zu erkennen, dass an den fünften und sechsten Hebeln 100, 104 die dritten und vierten Schnittstellen 42, 44 ausgebildet sind. Über die dritten und vierten Schnittstellen 42, 44 werden allerdings nicht die Hebel 100, 104 angetrieben, sondern die Hebel der zweiten Lenkeranordnung 18, die in Figur 9 allerdings nicht gezeigt sind. Die zweite Lenkeranordnung 18 liegt mit Bezug auf Figur 9 in der Zeichenebene unterhalb der ersten Lenkeranordnung 16 und ist identisch ausgebildet. Bei der zweiten Lenkeranordnung 18 werden allerdings nicht die mit Bezug auf Figur 9 links angeordneten Hebel 20, 22 angetrieben, sondern die parallel und unterhalb zu den fünften, sechsten Hebeln 104 angeordneten, korrespondierenden Hebel. Auf diese Weise lassen sich sowohl die erste als auch die zweite Lenkeranordnung 16, 18 von einer Seite des Manipulators 10 aus bedienen, während bei dem ersten Ausführungsbeispiel, wie in Figur 1 gezeigt, die Antriebseinheit zunächst von oben mit der ersten Lenkeranordnung 16 und dann von unten mit der zweiten Lenkeranordnung 18 zu koppeln ist. Hierdurch ist die Bedienung weiter vereinfacht.

Ein weiteres Merkmal, dass in Figur 9 zu sehen ist, ist eine Anzeigeeinrichtung 130. Die Anzeigeeinrichtung 130 umfasst in dieser Ausführungsform vier LED-Ringe 131, 132, 133, 134, die koaxial um die Rotationsachsen der Hebel 20, 22, 100, 104 und um die Schnittstellen 38, 40, 42, 44 angeordnet sind. Über diese LED-Ringe 131, 132, 133, 134, wird einem Bediener angezeigt, an welcher der Schnittstellen 38, 40, 42, 44 er die handgehaltene Antriebseinheit 50 einsetzen soll, um die Pose der ersten und zweiten Lenkeranordnung 16, 18 und somit die Position der Gelenkpunkte 17, 19 zu verstellen. Es kann auch vorgesehen sein, dass vor, und/oder während des Übertragens der Stellbewegung die LED-Ringe 131, 132, 133, 134, eine andere Farbe annehmen, oder eine umlaufende Beleuchtung einzelner LED-Elemente der LED-Ringe 131, 132, 133, 134, erfolgt, um die Rotationsrichtung der Hebel 20, 22, 100, 104 anzuzeigen. Es kann auch vorgesehen sein, dass die LED-Ringe 131, 132, 133, 134 das Erreichen der Soll-Position durch Aufleuchten in einer weiteren Farbe, durch Blinken oder anderweitige Aufleuchten anzeigen. Ebenso ist es möglich, dass über die LED-Ringe 131, 132, 133, 134 das Erreichen und/oder Überschreiten eines vorbestimmten Schwellwerts in Bezug auf eine Kraft, die auf die Gelenkpunkte 17, 19, beziehungsweise ein Tool Center Point wirkt, angezeigt wird. Hierzu sind die LED-Ringe 131, 132, 133, 134 vorzugsweise mit den Verarbeitungseinheiten VE (vgl. Fig. 5) verbunden.

Figur 10 illustriert ein drittes Ausführungsbeispiel des Manipulators 10, beziehungsweise insbesondere ein drittes Ausführungsbeispiel der ersten Lenkeranordnung 16. Wiederum sind gleiche und ähnliche Elemente mit gleichen Bezugszeichen versehen und insofern wird vollumfänglich auf die obige Beschreibung Bezug genommen. Insbesondere ist die Kinematik der Lenkeranordnung 16 gemäß diesem Ausführungsbeispiel (Fig. 10) identisch zu der Kinematik der Lenkeranordnung 16 des zweiten Ausführungsbeispiels (Fig. 9) ausgebildet. Insofern wird im Folgenden insbesondere auf die Unterschiede zwischen den Ausführungsbeispielen gemäß Figur 9 und 10 eingegangen.

Der wesentliche Unterschied zwischen diesen beiden Ausführungsbeispielen liegt in der Anzeigeeinrichtung 130. Während diese bei dem Ausführungsbeispiel gemäß Figur 9 vier LED-Ringe 131, 132, 133, 134 umfasst, ist bei dem Ausführungsbeispiel in Figur 10 eine rein mechanische Anzeigeeinrichtung 130 vorgesehen. Der Manipulator 10 gemäß dem Ausführungsbeispiel aus Figur 10 weist keinerlei elektronische Bausteine auf und ist somit dafür geeignet, in einem Computertomographen eingesetzt zu werden. Die mechanische Anzeigeeinrichtung 130 umfasst Markierungen 140, 141, 142, 143, die mit den entsprechenden Schnittstellen 38, 40, 42, 44 gekoppelt sind. Die Markierungen 140, 141, 142, 143 zeigen eine Drehstellung der entsprechenden Hebel an und sind optisch erfassbar.

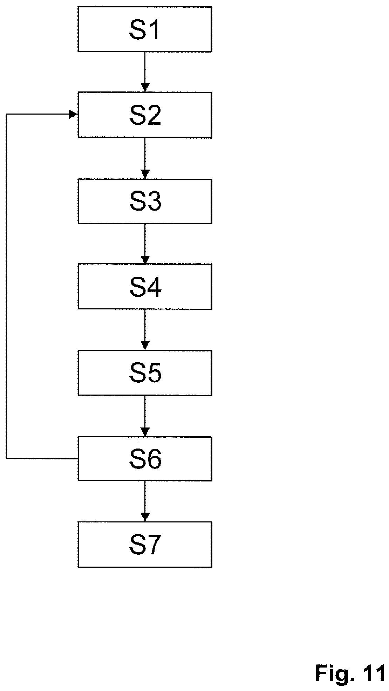

Gemäß Figur 11 ist schließlich ein Ausführungsbeispiel für ein Verfahren zum Einstellen einer Soll-Position von zwei Gelenkpunkten 17, 19 eines passiven chirurgischen Manipulators 10 nach einer der vorstehend beschriebenen Ausführungsformen dargestellt.

Insbesondere bezieht sich das in Figur 11 dargestellte Verfahren auf das zweite Ausführungsbeispiel gemäß Figur 9. Es soll aber verstanden werden, dass eine Anzeigeeinrichtung 130, wie sie im zweiten Ausführungsbeispiel gemäß Figur 9 dargestellt ist, auch bei dem ersten Ausführungsbeispiel gemäß der Figuren 1 bis 8 vorgesehen sein kann. Bei dem Ausführungsbeispiel gemäß Figur 1 wären dann die LED-Ringe 131, 132, 133, 134 auf entsprechend gegenüberliegenden Seiten des Rahmes 14 angeordnet.

In dem in Figur 11 dargestellten Verfahren ist bereits eine Soll-Pose an einer Steuereinheit des Manipulators 10, oder einer externen Steuereinheit empfangen worden. In Schritt S1 findet dann eine Berechnung der Ziel-Pose der ersten und zweiten Lenkeranordnungen 16, 18 und der sequenziellen Bewegungsreihenfolge und Bewegungsprofile der einzelnen Freiheitsgrade der Lenkeranordnungen 16, 18 statt. Das heißt, es wird berechnet, wie die einzelnen Hebel 20, 22, 100, 104 zu rotieren sind, um die Gelenkpunkte 17, 19 in die gewünschte Position zu bringen.

In Schritt S2 wird ein erster LED-Ring 131 abweichend zu den übrigen LED-Ringen 132, 133, 134 beleuchtet, um anzuzeigen, dass die Antriebseinheit 50 mit der diesem LED-Ring 131 zugeordneten Schnittstelle 38 zu koppeln ist. Hierzu wird ein entsprechendes Signal von der Steuereinheit an den entsprechenden LED-Ring 131 gesendet.

Im Schritt S3 koppelt ein Bediener die handgehaltene Antriebseinheit 50 mit der durch den LED-Ring 131 angezeigten Schnittstelle 38. Die handgehaltene Antriebseinheit 50 registriert die Kopplung und überträgt sodann die vorbestimmte Stellbewegung über die Schnittstelle 38 auf die entsprechende zugeordnete erste Lenkeranordnung 16. Es kann auch vorgesehen sein, dass die handgehaltene Antriebseinheit 50 nicht automatisch das Kuppeln mit der ersten Schnittstelle 38 registriert; in diesem Fall schaltet ein Bediener über das Anzeige- und Bedienpaneel 80 der handgehaltenen Antriebseinheit 50 diese frei, um die Stellbewegung auf die Schnittstelle 38 zu übertragen. Hierzu kann ein Menü auf dem Anzeigebedienpaneel angezeigt werden.

Durch Einschalten des Rotors 62 der handgehaltenen Antriebseinheit 50 wird in Schritt 4 die Stellbewegung übertragen und zwar solange, bis die gewünschte Soll-Position des entsprechenden Freiheitsgrads erreicht ist.

In Schritt 5 wird nach Erreichen dieser Ziel-Position der Motor 62 durch die Steuereinheit 64 der handgehaltenen Antriebseinheit 50 abgeschaltet und der LED-Ring 131 der entsprechenden Schnittstelle 38 wechselt die Farbe, um das Erreichen der Soll-Position anzuzeigen.

In Schritt S6 findet dann die Entscheidung statt, ob ein weiterer Freiheitsgrad verstellt werden soll. Für diesen Fall ist, wie durch den zu Schritt 2 führenden Pfeil angezeigt, ein weiterer LED-Ring, beispielsweise LED-Ring 132, beleuchtet, um das Koppeln der handgehaltenen Antriebseinheit 50 mit der entsprechend dann zugeordneten Schnittstelle 40 anzuzeigen. Die Schritte S2, S3, S4 und S5 folgen dann analog mit Bezug auf die jeweilige Schnittstelle 38, 40, 42, 44. Falls in Schritte bestimmt wird, dass keine weitere Verstellung erforderlich ist, wird in Schritt 7 festgestellt, dass das Verstellen abgeschlossen ist. Dies kann angezeigt werden durch ein einfaches Aufleuchten aller LED-Ringe 131, 132, 133, 134, oder durch eine andere Art der Anzeige, wie beispielsweise ein Wechseln der Farbe der LED-Ringe oder auch durch ein akustisches Signal, und/oder durch das Anzeigebedienpaneel 80.

## Patentansprüche

1. Passiver chirurgischer Manipulator (10) zum Halten und Positionieren eines chirurgischen Instruments (12), mit
einem Rahmen (14),
einer mindestens einen Lenker (20, 22, 24, 26) umfassenden ersten Lenkeranordnung (16), welche den Rahmen (14) mit einem dem Instrument (12) zugeordneten ersten Gelenkpunkt (17) gelenkig verbindet, und
einer mindestens einen Lenker umfassenden zweiten Lenkeranordnung (18), welche den Rahmen (14) mit einem dem Instrument (12) zugeordneten zweiten Gelenkpunkt (19) gelenkig verbindet,
wobei die beiden Lenkeranordnungen (16, 18) so ausgebildet sind, dass der erste Gelenkpunkt (17) in einer ersten Bewegungsebene und der zweite Gelenkpunkt (19) in einer zweiten Bewegungsebene bewegbar ist,
wobei die erste Lenkeranordnung (16) an ersten und zweiten Stelleinrichtungen (28, 30) mit dem Rahmen (14) verbunden ist, und die zweite Lenkeranordnung an dritten und vierten Stelleinrichtungen (32, 34) mit dem Rahmen (14) verbunden ist,
**dadurch gekennzeichnet, dass** die erste Stelleinrichtung (28) eine erste Schnittstelle (38), die zweite Stelleinrichtung (30) eine zweite Schnittstelle (40), die dritte Stelleinrichtung (32) eine dritte Schnittstelle (42) und die vierte Stelleinrichtung (34) eine vierte Schnittstelle (44) zur temporären Aufnahme einer korrespondierenden Schnittstelle (70) einer handgehaltenen Antriebseinheit (50) aufweist,
sodass die handgehaltene Antriebseinheit (50) temporär und sequenziell mit den ersten, zweiten, dritten und vierten Schnittstellen (38, 40, 42, 44) zum Übertragen einer Stellbewegung koppelbar ist, um die ersten und zweiten Gelenkpunkte (17, 19) zu bewegen und in einer vorbestimmten Soll-Position zu positionieren.

2. Manipulator nach Anspruch 1, wobei die Schnittstellen (38, 40, 42, 44) räumlich voneinander getrennt sind.

3. Manipulator nach einem der vorstehenden Ansprüche, wobei die Schnittstellen (38, 40, 42, 44) an einer gemeinsamen Kupplungsausnehmung (54) ausgebildet sind.

4. Manipulator nach einem der vorstehenden Ansprüche, wobei die Schnittstellen (38, 40, 42, 44) für eine formschlüssige Aufnahme der handgehaltenen Antriebseinheit (50) und zur formschlüssigen Übertragung der Stellbewegung ausgebildet sind; oder die Schnittstellen (38, 40, 42, 44) für eine formschlüssige Aufnahme der handgehaltenen Antriebseinheit (50) und zur kraftschlüssigen Übertragung der Stellbewegung ausgebildet sind, wobei jede Schnittstelle (38, 40, 42, 44) vorzugsweise ein ferromagnetisches Kupplungselement aufweist.

5. Manipulator nach einem der vorstehenden Ansprüche, aufweisend wenigstens einen Sensor (S) zur Ermittlung einer Kraft, die auf den ersten und/oder zweiten Gelenkpunkt (17, 19) wirkt.

6. Manipulator nach einem der vorstehenden Ansprüche, aufweisend eine Steuereinheit mit Prozessor und Softwaremitteln, die dazu ausgebildet ist, basierend auf einer vorbestimmten Soll-Stellung des ersten und zweiten Gelenkpunkts (17, 19) eine an jeder Schnittstelle (38, 40, 42, 44) erforderliche Stellbewegung zum Erreichen der Soll-Stellung zu ermitteln, wobei die Steuereinheit vorzugsweise dazu eingerichtet ist, Signale, die die ermittelten Stellbewegungen repräsentieren, an der handgehaltenen Antriebseinheit (50) oder einem Peripheriegerät bereitzustellen, wobei vorzugsweise jede Schnittstelle (38, 40, 42, 33) eine Anzeigeeinheit (130, 131, 132, 133, 134, 141, 142, 143, 144) aufweist, die dazu eingerichtet ist, ein Erreichen der Soll-Stellung anzuzeigen, wobei die Anzeigeeinheiten (130, 131, 132, 133, 134, 141, 142, 143, 144) vorzugsweise dazu eingerichtet sind, eine Sequenz der Kupplung mit der handgehaltenen Antriebseinheit (50) anzuzeigen.

7. Manipulator nach einem der vorstehenden Ansprüche, wobei an den Schnittstellen (38, 40, 42, 44) Markierungen (141, 142, 143, 144) angeordnet sind, die durch eine entsprechende Leseeinheit abtastbar sind, zur Ermittlung einer Stellung der ersten und zweiten Gelenkpunkte (17, 19).

8. Manipulator nach einem der vorstehenden Ansprüche, wobei der Manipulator (10) vollständig aus einem Kunststoff, vorzugsweise mittels eines Rapid-Prototyping-Verfahrens, gebildet ist.

9. Chirurgisches System umfassend wenigstens einen Manipulator (10) nach einem der Ansprüche 1 bis 8 und eine handgehaltene Antriebseinheit (50) zur Verwendung mit einem passiven chirurgischen Manipulator (10) nach einem der vorstehenden Ansprüche 1 bis 8, mit
einem Gehäuse (60), in dem ein Antriebsmotor (62) und eine Steuereinheit (64) mit einem Prozessor und Softwaremitteln angeordnet sind, und
einer Schnittstelle (70) zum temporären und sequenziellen Kuppeln mit einer korrespondierenden Schnittstelle (38, 40, 42, 44) des Manipulators (10) und zum Übertragen einer Stellbewegung auf den Manipulator (10),
wobei die Steuereinheit (64) dazu eingerichtet ist den Antriebsmotor (62) derart zu steuern, dass eine Stellbewegung übertragen wird, die dazu geeignet ist, die ersten und zweiten Gelenkpunkte (17, 19) zu bewegen und in einer vorbestimmten Soll-Position zu positionieren.

10. System nach Anspruch 9, wobei die Schnittstelle (70) der Antriebseinheit eine Abtriebswelle (68) mit einem elektronischen Kontakt (72) zur Übertragung von Signalen aufweist, und wobei die Abtriebswelle (70) vorzugsweise Kupplungsmittel zum Kuppeln mit den Schnittstellen des Manipulators (10) aufweist, wobei die Abtriebswelle (68) vorzugsweise für jede Schnittstelle (38, 40, 42, 44) des Manipulators (10) ein separates Kupplungsmittel (84a, 84b, 84c, 84d) aufweist.

11. System nach einem der Ansprüche 9 oder 10, wobei die Steuereinheit (64) der Antriebseinheit dazu ausgebildet ist, eine vorbestimmte Soll-Position des ersten und zweiten Gelenkpunkts (17, 19) zu empfangen, wobei die Steuereinheit (64) vorzugsweise dazu ausgebildet ist, basierend auf der vorbestimmten Soll-Position des ersten und zweiten Gelenkpunkts (17, 19) eine an jeder Schnittstelle (38, 40, 42, 44) erforderliche Stellbewegung zum Erreichen der Soll-Position zu ermitteln.

12. System nach einem der Ansprüche 9 bis 11, wobei die Antriebseinheit wenigstens eine Infrarot-Strahlungsquelle aufweist, insbesondere IR-LED, zum Senden von Signalen an ein chirurgisches Navigationssystem.

13. System nach einem der Ansprüche 9 bis 12, wobei die Antriebseinheit wenigstens einen Sensor zum ermitteln eines an der Schnittstelle (70) der Antriebseinheit wirkenden mechanischen Widerstands aufweist, wobei der Sensor mit der Steuereinheit (64) verbunden ist, und die Steuereinheit (64) dazu eingerichtet ist die erfassten Daten zu verarbeiten und/oder an einem Peripheriegerät bereitzustellen, wobei das Verarbeiten vorzugsweise umfasst: Ermitteln von auf den ersten und zweiten Gelenkpunkt (17, 19) des Manipulators (10) wirkenden Kräften.

14. Nicht-chirurgisches Verfahren zum Einstellen einer Soll-Position von zwei Gelenkpunkten (17, 19) eines passiven chirurgischen Manipulators, insbesondere einem Manipulator (10) nach einem der Ansprüche 1 bis 8, mittels einer handgehaltenen Antriebseinheit, oder eines Systems nach einem der Ansprüche 9 bis 13, mit den Schritten:
- Ermitteln einer Ist-Position der zwei Gelenkpunkte (17, 19);
- Ermitteln einer Soll-Position der zwei Gelenkpunkte (17, 19);
- Ermitteln (S1) von Stellbewegungen für jede Lenkeranordnung (16, 18) des Manipulators (10), und
- Übertragen (S3) der Stellbewegung mittels der handgehaltenen Antriebseinheit (50) an dafür vorgesehenen Schnittstellen (38, 40, 42, 44) des Manipulators (10), durch temporäres und sequenzielles Kuppeln der handgehaltenen Antriebseinheit (50) mit den entsprechenden Schnittstellen (38, 40, 42, 44) des Manipulators (10).

## Claims

1. A passive surgical manipulator (10) for holding and positioning a surgical instrument (12), having
a frame (14),
a first suspension arm arrangement (16) comprising at least one suspension arm (20, 22, 24, 26) and connecting the frame (14) to a first joint (17) associated with the instrument (12) in an articulated manner, and
a second suspension arm arrangement (18) comprising at least one suspension arm and connecting the frame (14) to a second joint (19) associated with the instrument (12) in an articulated manner,
the two suspension arm arrangements (16, 18) being implemented so that the first joint (17) is displaceable in a first motion plane and the second joint (19) is displaceable in a second motion plane,
the first suspension arm arrangement (16) being connected to the frame (14) at first and second actuating devices (28, 30) and the second suspension arm arrangement being connected to the frame (14) at third and fourth actuating devices (32, 34),
**characterized in that** the first actuating device (28) comprises a first interface (38), the second actuating device (30) comprises a second interface (40), the third actuating device (32) comprises a third interface (42), and the fourth actuating device (34) comprises a fourth interface (44) for temporarily receiving a corresponding interface (70) of a handheld drive unit (50),
so that the handheld drive unit (50) can be coupled temporarily and sequentially to the first, second, third, and fourth interfaces (38, 40, 42, 44) for transferring an actuating motion in order to displace the first and second joints (17, 19) and position the same in a predetermined, specified position.

2. The manipulator according to claim 1, wherein the interfaces (38, 40, 42, 44) are spatially separated from each other.

3. The manipulator according to any one of the preceding claims, wherein the interfaces (38, 40, 42, 44) are implemented on a common coupling cutout (54).

4. The manipulator according to any one of the preceding claims, wherein the interfaces (38, 40, 42, 44) are implemented for positively receiving the handheld drive unit (50) and for positively transmitting the actuating motion; or
the interfaces (38, 40, 42, 44) are implemented for positively receiving the handheld drive unit (50) and for transmitting the actuating motion by means of a force fit, wherein each interface (38, 40, 42, 44) preferably comprises a ferromagnetic coupling element.

5. The manipulator according to one of the preceding claims, comprising at least one sensor (S) for determining the force acting on the first and/or the second joint (17, 19).

6. The manipulator according to any one of the preceding claims, comprising a control unit having a processor and software means implemented form determining an actuating motion required at each interface (38, 40, 42, 44) for reaching the specified setting, based on a predetermined specified setting for the first and second joints (17, 19), wherein the control unit preferably is set up for providing signals representing the determined actuating motions at the handheld drive unit (50) or a peripheral device, wherein preferably each interface (38, 40, 42, 33) comprises an indicator unit (130, 131, 132, 133, 134, 141, 142, 143, 144) set up for indicating that the specified setting has been reached, wherein the indicator units (130, 131, 132, 133, 134, 141, 142, 143, 144) preferably are set up for indicating a sequence of coupling to the handheld drive unit (50).

7. The manipulator according to any one of the preceding claims, wherein markings (141, 142, 143, 144) readable by a corresponding reading unit are disposed at the interfaces (38, 40, 42, 44) for determining a setting of the first and second joints (17, 19).

8. The manipulator according to any one of the preceding claims, wherein the manipulator (10) is made entirely of plastic, preferably using a rapid-prototyping method.

9. A surgical system comprising at least one manipulator (10) according to any one of the claims 1 through 8 and a hand-held drive unit (50) for using with a passive surgical manipulator (10) according to any one of the preceding claims 1 through 8, having
a housing (60) in which a drive motor (62) and a control unit (64) having a processor and software means are disposed, and
an interface (70) for temporarily and sequentially coupling to a corresponding interface (38, 40, 42, 44) of the manipulator (10) and for transmitting an actuating motion to the manipulator (10),
wherein the control unit (64) is set up for controlling the drive motor (62) such that an actuating motion is transmitted and is suitable for displacing the first and second joints (17, 19) and positioning the same in a predetermined specified position.

10. The system according to claim 9, wherein the interface (70) of the drive unit comprises an output shaft (68), comprising an electronic contact (72) for transmitting signals, and wherein the output shaft (70) preferably comprises coupling means for coupling to the interfaces of the manipulator (10), wherein the output shaft (68) preferably comprises a separate coupling means (84a, 84b, 84c, 84d) for each interface (38, 40, 42, 44) of the manipulator (10).

11. The system according to any one of the claims 9 or 10, wherein the control unit (64) of the drive unit is implemented for receiving a predetermined specified position of the first and second joints (17, 19), wherein the control unit (64) preferably is implemented for determining an actuating motion required at each interface (38, 40, 42, 44) for reaching the specified position based on the predetermined specified position of the first and second joints (17, 19).

12. The system according to any one of the claims 9 through 11, wherein the drive unit comprises at least one infrared radiation source, particularly an IR LED, for transmitting signals to a surgical navigation system.

13. The system according to any one of the claims 9 through 12, wherein the drive unit comprises at least one sensor for determining a mechanical resistance acting at the interface (70) of the drive unit, wherein the sensor is connected to the control unit (64) and the control unit (64) is set up for processing the captured data and/or for providing the same to a peripheral device, wherein the processing preferably comprises: determining forces acting on the first and second joints (17, 19) of the manipulator (10).

14. A non-surgical method for setting a specified position of two joints (17, 19) of a passive surgical manipulator, particularly a manipulator (10) according to any one of the claims 1 through 8, by means of a handheld drive unit, or a system according to any one of the claims 9 through 13, having the steps:
- determining a current position of the two joints (17, 19);
- determining a specified position of the two joints (17, 19);
- determining (S1) actuating motions for each suspension arm arrangement (16, 18) of the manipulator (10), and
- transferring (S3) the actuating motion by means of the handheld drive unit (50) at interfaces (38, 40, 42, 44) of the manipulator (10) provided for this purpose by temporarily and sequentially coupling the handheld drive unit (50) to the corresponding interfaces (38, 40, 42, 44) of the manipulator (10).

## Revendications

1. Manipulateur chirurgical passif (10) destiné à tenir et à positionner un instrument chirurgical (12), comprenant
un cadre (14);
un premier arrangement de bras oscillant (16) comprenant au moins un bras oscillant (20, 22, 24, 26), qui relie de manière articulée le cadre (14) à un premier point d'articulation (17) associé à l'instrument (12), et
un deuxième arrangement de bras oscillant (18) comprenant au moins un bras oscillant, qui relie de manière articulée le cadre (14) à un deuxième point d'articulation (19) associé à l'instrument (12),
dans lequel les deux arrangements de bras oscillant (16, 18) sont conçus de telle sorte que le premier point d'articulation (17) est mobile dans un premier plan de mouvement et le deuxième point d'articulation (19) est mobile dans un deuxième plan de mouvement,
dans lequel le premier arrangement de bras oscillant (16) est relié au cadre (14) au niveau de premier et deuxième dispositifs de positionnement (28, 30), et le deuxième arrangement de bras oscillant est relié au cadre (14) au niveau de troisième et quatrième dispositifs de positionnement (32, 34),
**caractérisé en ce que** le premier dispositif de positionnement (28) comprend une première interface (38), le deuxième dispositif de positionnement (30) comprend une deuxième interface (40), le troisième dispositif de positionnement (32) comprend une troisième interface (42) et le quatrième dispositif de positionnement (34) comprend une quatrième interface (44) en vue de recevoir temporairement une interface correspondante (70) d'une unité d'entraînement (50) tenue à la main,
de sorte que l'unité d'entraînement (50) tenue à la main peut être couplée temporairement et séquentiellement aux première, deuxième, troisième et quatrième interfaces (38, 40, 42, 44) en vue de transmettre un mouvement de positionnement afin de déplacer les premier et deuxième points d'articulation (17, 19) et de les positionner dans une position nominale prédéterminée.

2. Manipulateur selon la revendication 1, dans lequel les interfaces (38, 40, 42, 44) sont séparées spatialement les unes des autres.

3. Manipulateur selon l'une des revendications précédentes, dans lequel les interfaces (38, 40, 42, 44) sont formées sur un évidement d'accouplement commun (54).

4. Manipulateur selon l'une des revendications précédentes, dans lequel les interfaces (38, 40, 42, 44) sont réalisées pour une réception par complémentarité de formes de l'unité d'entraînement (50) tenue à la main et pour la transmission par complémentarité de formes du mouvement de positionnement; ou les interfaces (38, 40, 42, 44) sont réalisées pour une réception par complémentarité de formes de l'unité d'entraînement (50) tenue à la main et pour la transmission par friction du mouvement de positionnement, chaque interface (38, 40, 42, 44) possédant de préférence un élément d'accouplement ferromagnétique.

5. Manipulateur selon l'une des revendications précédentes, possédant au moins un capteur (S) destiné à déterminer une force agissant sur le premier et/ou le deuxième point d'articulation (17, 19).

6. Manipulateur selon l'une des revendications précédentes, possédant une unité de commande avec processeur et moyens logiciels, qui est conçue pour déterminer, sur la base d'une position nominale prédéterminée du premier et du deuxième point d'articulation (17, 19), un mouvement de positionnement nécessaire au niveau de chaque interface (38, 40, 42, 44) pour atteindre la position nominale, l'unité de commande étant de préférence configurée pour fournir des signaux, qui représentent les mouvements de positionnement déterminés, à l'unité d'entraînement tenue à la main (50) ou à un appareil périphérique, chaque interface (38, 40, 42, 33) possédant de préférence une unité d'affichage (130, 131, 132, 133, 134, 141, 142, 143, 144) qui est configurée pour indiquer que la position nominale est atteinte, les unités d'affichage (130, 131, 132, 133, 134, 141, 142, 143, 144) étant de préférence configurées pour indiquer une séquence de couplage avec l'unité d'entraînement tenue à la main (50).

7. Manipulateur selon l'une des revendications précédentes, dans lequel des marquages (141, 142, 143, 144) sont disposés sur les interfaces (38, 40, 42, 44), qui peuvent être balayés par une unité de lecture correspondante afin de déterminer une position des premier et deuxième points d'articulation (17, 19).

8. Manipulateur selon l'une des revendications précédentes, dans lequel le manipulateur (10) est entièrement formé d'une matière plastique, de préférence au moyen d'un procédé de prototypage rapide.

9. Système chirurgical comprenant au moins un manipulateur (10) selon l'une quelconque des revendications 1 à 8 et une unité d'entraînement tenue à la main (50) destinée à être utilisée avec un manipulateur chirurgical passif (10) selon l'une quelconque des revendications 1 à 8 précédentes, comprenant
un boîtier (60) dans lequel sont disposés un moteur d'entraînement (62) et une unité de commande (64) avec un processeur et des moyens logiciels, et
une interface (70) destinée au couplage temporaire et séquentiel avec une interface correspondante (38, 40, 42, 44) du manipulateur (10) et à la transmission d'un mouvement de positionnement au manipulateur (10),
l'unité de commande (64) étant configurée pour commander le moteur d'entraînement (62) de telle sorte qu'un mouvement de positionnement soit transmis, lequel est approprié pour déplacer les premier et deuxième points d'articulation (17, 19) et les positionner dans une position nominale prédéterminée.

10. Système selon la revendication 9, dans lequel l'interface (70) de l'unité d'entraînement comprend un arbre de sortie (68) pourvu d'un contact électronique (72) destiné à la transmission de signaux, et dans lequel l'arbre de sortie (70) comprend de préférence des moyens d'accouplement destinés à l'accouplement avec les interfaces du manipulateur (10), l'arbre de sortie (68) comprenant de préférence un moyen d'accouplement (84a, 84b, 84c, 84d) séparé pour chaque interface (38, 40, 42, 44) du manipulateur (10).

11. Système selon l'une quelconque des revendications 9 ou 10, dans lequel l'unité de commande (64) de l'unité d'entraînement est conçue pour recevoir une position nominale prédéterminée des premier et deuxième points d'articulation (17, 19), l'unité de commande (64) étant de préférence configurée pour déterminer, sur la base de la position nominale prédéterminée des premier et deuxième points d'articulation (17, 19), un mouvement de positionnement nécessaire au niveau de chaque interface (38, 40, 42, 44) pour atteindre la position nominale.

12. Système selon l'une quelconque des revendications 9 à 11, dans lequel l'unité d'entraînement comprend au moins une source de rayonnement infrarouge, en particulier une DEL IR, servant à envoyer des signaux à un système de navigation chirurgicale.

13. Système selon l'une quelconque des revendications 9 à 12, dans lequel l'unité d'entraînement possède au moins un capteur destiné à déterminer une résistance mécanique agissant sur l'interface (70) de l'unité d'entraînement, le capteur étant relié à l'unité de commande (64), et l'unité de commande (64) étant configurée pour traiterles données acquises et/ou les mettre à disposition sur un appareil périphérique, le traitement comprenant de préférence: déterminer des forces agissant sur les premier et deuxième points d'articulation (17, 19) du manipulateur (10).

14. Procédé non chirurgical de réglage d'une position nominale de deux points d'articulation (17, 19) d'un manipulateur chirurgical passif, en particulier d'un manipulateur (10) selon l'une quelconque des revendications 1 à 8, au moyen d'une unité d'entraînement tenue à la main, ou d'un système selon l'une quelconque des revendications 9 à 13, comprenant les étapes suivantes:
- déterminer une position réelle des deux points d'articulation (17, 19);
- déterminer une position nominale des deux points d'articulation (17, 19);
- déterminer (S1) des mouvements de positionnement pour chaque arrangement de bras oscillant (16, 18) du manipulateur (10), et
- transmettre (S3) le mouvement de positionnement au moyen de l'unité d'entraînement (50) tenue à la main à des interfaces (38, 40, 42, 44) du manipulateur (10) prévues à cet effet, par couplage temporaire et séquentiel de l'unité d'entraînement (50) tenue à la main avec les interfaces (38, 40, 42, 44) correspondantes du manipulateur (10).
